(19) Europäisches Patentamt
European Patent Office
Office européen des brevets

(11) Veröffentlichungsnummer: **0 017 800**
**B1**

(12) # EUROPÄISCHE PATENTSCHRIFT

(45) Veröffentlichungstag der Patentschrift: **22.12.82**

(51) Int. Cl.³: **C 07 C 175/00**

(21) Anmeldenummer: **80101545.4**

(22) Anmeldetag: **24.03.80**

(54) **Verfahren zur Herstellung von Cyclohexenderivaten sowie ein Zwischenprodukt in diesem Verfahren.**

(30) Priorität: **27.03.79 US 24296**

(43) Veröffentlichungstag der Anmeldung: **29.10.80 Patentblatt 80/22**

(45) Bekanntmachung des Hinweises auf die Patenterteilung: **22.12.82 Patentblatt 82/51**

(84) Benannte Vertragsstaaten: **AT BE CH DE FR GB IT NL**

(56) Entgegenhaltungen:
**US-A-4 045 476**
**US-A-4 098 827**

(73) Patentinhaber: **F. HOFFMANN-LA ROCHE & CO. *Aktiengesellschaft, CH-4002 Basel (CH)***

(72) Erfinder: **Hengartner, Urs, Dr., Binzenstrasse 28, CH-4058 Basel (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

**EP 0 017 800 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von Cyclohexenderivaten sowie ein Zwischenprodukt in diesem Verfahren

Die vorliegende Erfindung betrifft ein neues Verfahren zur Herstellung von Zwischenprodukten in der Totalsynthese von Canthaxanthin. Die Erfindung betrifft ferner eine neue Verbindung in diesem Verfahren.

Das erfindungsgemässe Verfahren ist dadurch gekennzeichnet, dass man die Verbindung der Formel

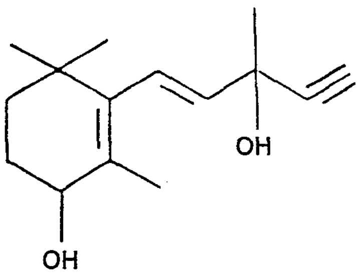

I

mit einem Oxidationsmittel umsetzt und dass man, gewünschtenfalls, die so erhaltene Verbindung der Formel

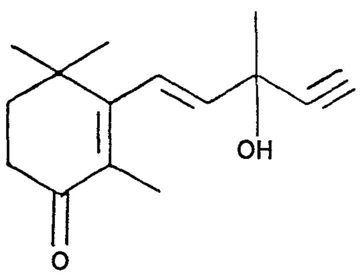

II

selektiv hydriert zur Verbindung der Formel

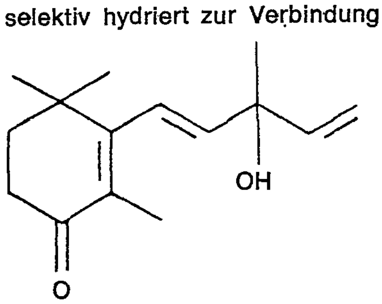

III

Die Verbindung der Formel II ist neu und Teil der vorliegenden Erfindung.

Die Verbindung der Formel III ist bekannt. Gemäss US-Patent Nr. 4 098 827 wurde die Verbindung der Formel III bisher aus der Verbindung der Formel I über das Zwischenprodukt der Formel

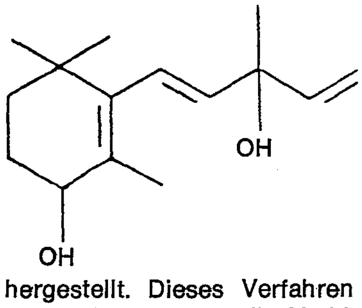

IV

hergestellt. Dieses Verfahren hat jedoch den Nachteil, dass man die Verbindung der Formel III als unreines Gemisch erhält. Da die Verbindung der Formel III ein Zwischenprodukt für den bekannten Lebensmittelfarbstoff Canthaxanthin ist, der in Lebensmitteln in reiner Form verwendet werden muss, ist es wichtig, dass das Zwischenprodukt im möglichst reiner Form hergestellt wird. Deshalb muss die Verbindung der Formel III, wenn sie über das Zwischenprodukt der Formel IV hergestellt wird, mit üblichen Mitteln gereinigt werden, bevor sie in der weiteren Synthese von Canthaxanthin eingesetzt werden kann.

Es wurde nun überraschenderweise gefunden, dass die Verbindung der Formel III ohne weitere Reinigung in einer Reinheit von mindestens 96% erhalten wird, wenn sie aus der Verbindung der Formel I über das Zwischenprodukt der Formel II hergestellt wird. Tatsächlich wird diese Reinheit unabhängig von der Reinheit des verwendeten Ausgangsmaterials der Formel I erhalten. Das erfindungsgemässe Verfahren ermöglicht daher, ohne teure und zeitraubende Reinigungsverfahren, die Herstellung eines reinen Zwischenproduktes, selbst wenn dabei unreine Ausgangsmaterialien verwendet werden.

Gemäss dem erfindungsgemässen Verfahren wird die Verbindung der Formel I mit einem Oxidationsmittel zur Verbindung der Formel II umgesetzt. Die Umsetzung kann mit irgendeinem üblichen Oxidationsmittel erfolgen. Zu den bevorzugten Oxidationsmitteln gehören Mangandioxid, Chromtrioxid und Chromat-Oxidationsmittel, wie das Jones-Reagens, sowie Aceton in Gegenwart eines Aluminiumalkoxids. Die Umsetzung kann bei irgendwelchen, in solchen Oxidationen üblicherweise angewendeten Bedingungen durchgeführt werden.

Besonders bevorzugt ist die Oxidation der Verbindung der Formel I zur Verbindung der Formel II mit Aceton in Gegenwart eines Aluminiumalkoxids. Dabei können irgendwelche üblichen Aluminiumalkoxide verwendet werden, wie Aluminium-nieder-alkoxide, d.h. solche worin die Alkoxygruppen 1 bis 7 Kohlenstoffatome enthalten. Bevorzugte Aluminiumalkoxide sind Aluminiumisopropoxid und Aluminium-t-butoxid. Das Aluminiumalkoxid kann in dieser Reaktion in katalytischen Mengen vorliegen, d.h. in Mengen von mindestens etwa 0,1 Molprozent der Menge der Verbindung der Formel I. Das Aluminiumalkoxid kann aber gewünschtenfalls auch in Mengen von etwa 100 Molprozenten, verglichen mit der Verbindung der Formel I, vorliegen, und auch ein grosser Überschuss beeinflusst die Reaktion nicht nachteilig. Aus Gründen der Wirtschaftlichkeit wird man jedoch das Aluminiumalkoxid bevorzugt in einer Menge von etwa 0,1 bis etwa 100 Molprozenten verwenden.

Im allgemeinen wird die Oxidation mit Aceton und einem Aluminiumalkoxid in Aceton als organisches Lösungsmittelmedium ausgeführt. Gewünschtenfalls kann das Reaktionsmedium

zusätzliche Lösungsmittel wie Benzol und Toluol enthalten; es kann aber tatsächlich irgendein inertes organisches Lösungsmittel im Gemisch mit Aceton verwendet werden. Anderseits kann die Reaktion auch in Aceton ohne Zusatz eines inerten organischen Lösungsmittels ausgeführt werden. Die Reaktion wird im allgemeinen bei der Rückflusstemperatur des Reaktionsgemisches durchgeführt.

Gemäss dem erfindungsgemässen Verfahren wird die Verbindung der Formel II als kristalline Substanz erhalten und kann leicht aus dem Reaktionsgemisch abgetrennt werden. Es können die üblichen Kristallisationsmethoden angewendet werden, und die Verbindung der Formel II kann leicht kristallisiert werden aus gebräuchlichen unpolaren Lösungsmitteln. Bevorzugte unpolare Lösungsmittel sind z. B. Hexan, Petroläther und dergleichen.

Die Umsetzung der Verbindung der Formel II zur Verbindung der Formel III durch teilweise Hydrierung der Verbindung der Formel II kann in an sich bekannter Weise erfolgen. Diese Umsetzung kann mit irgendeinem der Katalysatoren ausgeführt werden, die üblicherweise für die teilweise Hydrierung von Dreifachbindungen zu Doppelbindungen verwendet werden. Bevorzugte Katalysatoren sind die teilweise vergifteten Palladium-Katalysatoren (z. B. Lindlar-Katalysatoren), wie sie beispielsweise in Helvetica Chimica Acta 35, 446 (1952) beschrieben sind. Zur Durchführung dieser Reaktion wird die Verbindung der Formel II in einem inerten organischen Lösungsmittel gelöst. Es kann irgendeines der gebräuchlichen inerten organischen Lösungsmittel verwendet werden. Bevorzugte inerte organische Lösungsmittel sind Äthylacetat, Toluol, Petroläther, Methylenchlorid und dergleichen. Gewünschtenfalls können weitere gebräuchliche Katalysatorgifte, wie 1,2-Bis-(2--hydroxyäthylthio)äthan, verwendet werden, um den Katalysator weiter zu desaktivieren. Temperatur und Druck sind keine kritischen Aspekte in dieser Reaktion. Die Reaktion kann bei Raumtemperatur und Atmosphärendruck durchgeführt werden, gewünschtenfalls können aber höhere oder tiefere Temperaturen angewendet werden. Im allgemeinen wird eine Reaktionstemperatur von etwa 10°C bis etwa 70°C bevorzugt.

Gemäss dem erfindungsgemässen Verfahren wird die durch selektive Hydrierung hergestellte Verbindung der Formel III in mindestens 96%iger Reinheit erhalten.

Die Verbindung der Formel I ist bekannt und kann nach bekannten Methoden hergestellt werden.

Die Erfindung wird durch die folgenden Beispiele weiter veranschaulicht.

Beispiel 1

In einen 5-Liter-Dreihalskolben mit Rührer, Thermometer und Rückflusskühler wurden unter Stickstoff 255 g (1 Mol) 1-(3-Hydroxy-2,6,6--trimethyl-1-cyclohexen-1-yl)-3-methyl-1-penten--4-in-3-ol, 0,7 l Aceton, 1,2 l Toluol und 80 g (0,39 Mol) Aluminiumisopropoxid eingebracht. Die Mischung wurde gerührt und 2,5 Stunden zum Rückfluss erhitzt. Der gekühlte Kolbeninhalt wurde in ein Gemisch von 67 g (0,65 Mol) konzentrierter (96%) Schwefelsäure und 1,2 l Eiswasser gegossen und 5 Minuten gerührt. Die organische Phase wurde nacheinander mit 300 ml Salzwasser, 500 ml gesättigter wässriger Natriumbicarbonat-Lösung und 300 ml Salzwasser gewaschen. Die wässrige Phase und die Waschlösungen wurden in der gleichen Reihenfolge mit 400 ml Toluol extrahiert. Die vereinigten organischen Phasen wurden über Magnesiumsulfat getrocknet. Nach dem Einengen am Rotationsverdampfer (40°C/25 mmHg) wurde ein viskoses Öl (290 g) erhalten, das beim Stehenlassen bei Raumtemperatur auskristallisierte. Diese Substanz wurde in 350 ml warmen Toluol gelöst, und die Lösung auf Raumtemperatur abkühlen gelassen. Dann wurden unter Rühren 500 ml Hexan zugegeben und die klare, gelbe Lösung über Nacht bei Raumtemperatur gerührt. Das kristalline Material wurde genutscht, mit 400 ml Hexan/Toluol 3:1 gewaschen und am Vakuum getrocknet (bis zu konstantem Gewicht). Es wurden 147,9 g (63,7%) 1-(3-Oxo-2,-6,6-trimethyl-1-cyclohexen-1-yl)-3-methyl-1-penten-4-in-3-ol als schwach gelbliche Kristalle erhalten; Smp. 86-88°C. Die Mutterlauge wurde am Rotationsverdampfer eingeengt und das zurückbleibende Öl (89 g) bei 130-165°C/0,05 mmHg destilliert. Dabei wurden 69,8 g dunkelgelbes Destillat erhalten. Dieses wurde in 100 ml Toluol gelöst, 250 ml Hexan zugegeben und die erhaltene gelbe Lösung angeimpft und über Nacht bei Raumtemperatur gerührt. Der Niederschlag wurde abfiltriert, mit 75 ml Hexan/Toluol 3:1 gewaschen und am Vakuum getrocknet. Dabei wurden zusätzliche 17,9 g (7,7%) 1-(3-Oxo--2,6,6-trimethyl-1-cyclohexen-1-yl)-3-methyl-1--penten-4-in-3ol als weisse Kristalle erhalten; Smp. 86-88°C.

Beispiel 2

In einen 2-Liter-Dreihalskolben mit Rührer und Gaseinlass und -auslass wurden unter Stickstoff 500 ml Methylenchlorid und 8,25 g Lindlar-Katalysator eingebracht. Unter Rühren wurden eine Lösung von 84 mg 1,2-Bis-(2-hydroxyäthylthio)äthan in 17 ml Methylenchlorid, gefolgt von 10 ml Triäthylamin zugegeben. Anschliessend wurde eine Lösung von 164,7 g (0,709 Mol) 1-(3--Oxo-2,6,6-trimethyl-1-cyclohexen-1-yl)-3-methyl--1-penten-4-in-3-ol in 350 ml Methylenchlorid zugegeben und der Kolben mit Wasserstoff gespült. Die Mischung wurde dann bei Raumtemperatur und 790 mmHg hydriert, bis die Wasserstoffaufnahme beendet war (8 Stunden). Die Mischung wurde filtriert und das klare hellgelbe Filtrat am Rotationsverdampfer eingeengt zuerst 35°C/25 mmHg und dann 35°C/2 mmHg). Dabei wurden 167,2 g (100,7%) reines (über 96%iges) 1-(3-Oxo-2,6,6-trimethyl-1-cyclohexen--1-yl)-3-methyl-1,4-pentadien-3-ol als gelbes viskoses Öl erhalten.

**Patentansprüche**

für die Vertragsstaaten: BE, CH, DE, FR, GB, IT, NL

1. Verfahren zur Herstellung von Cyclohexenderivaten, dadurch gekennzeichnet, dass man die Verbindung der Formel

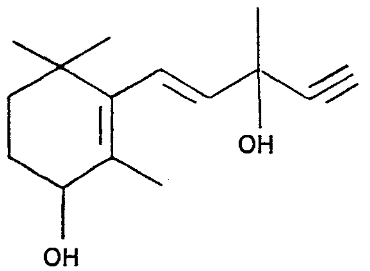

I

mit einem Oxidationsmittel umsetzt und dass man, gewünschtenfalls, die so erhaltene Verbindung der Formel

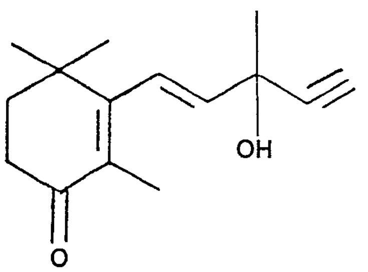

II

selektiv hydriert zur Verbindung der Formel

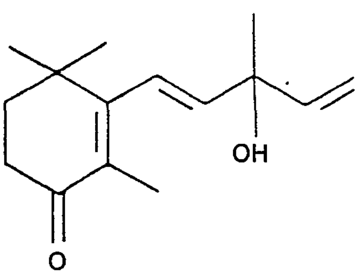

III

2. Verfahren gemäss Anspruch 1, dadurch gekennzeichnet, dass man als Oxidationsmittel Mangandioxid, Chromtrioxid, ein Chromat oder, vorzugsweise, Aceton in Gegenwart eines Aluminiumalkoxids verwendet.

3. Verfahren gemäss Anspruch 2, dadurch gekennzeichnet, dass man als Oxidationsmittel Aceton in Gegenwart von Aluminiumisopropoxid oder Aluminium-t-butoxid verwendet.

4. Verfahren gemäss einem der Ansprüche 1 bis 3, dadurch gekennzeichnet, dass man das Aluminiumalkoxid in einer Menge von etwa 0,1 bis etwa 100 Molprozent verwendet.

5. Verfahren gemäss einem der Ansprüche 1 bis 4, dadurch gekennzeichnet, dass man die Oxidation bei der Rückflusstemperatur des Reaktionsgemisches durchführt.

6. Verfahren gemäss einem der Ansprüche 1 bis 5, dadurch gekennzeichnet, dass man die Hydrierung mit einem teilweise vergifteten Palladium-Katalysator, vorzugsweise einem Lindlar-Katalysator durchführt.

7. Verfahren gemäss einem der Ansprüche 1 bis 6, dadurch gekennzeichnet, dass man die Hydrierung bei einer Temperatur von etwa 10°C bis etwa 70°C durchführt.

8. Verbindung der Formel

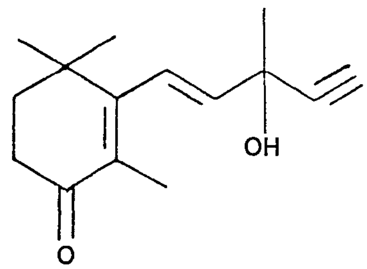

II

**Patentansprüche**

für den Vertragsstaat AT

1. Verfahren zur Herstellung von Cyclohexenderivaten, dadurch gekennzeichnet, dass man die Verbindung der Formel

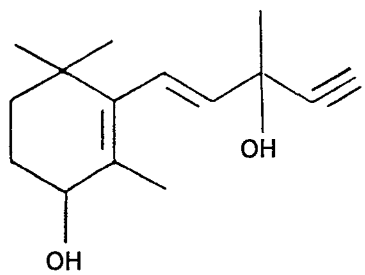

I

mit einem Oxidationsmittel umsetzt und dass man, gewünschtenfalls, die so erhaltene Verbindung der Formel

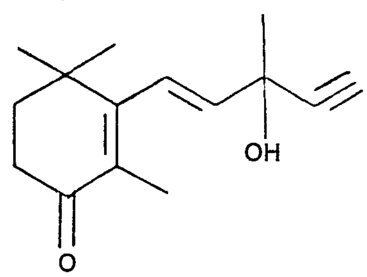

II

selektiv hydriert zur Verbindung der Formel

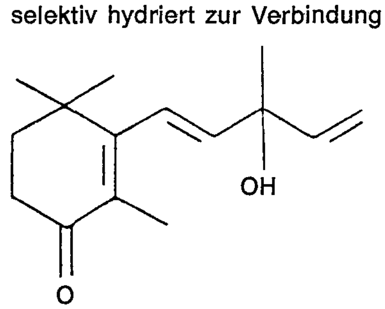

III

2. Verfahren gemäss Anspruch 1, dadurch gekennzeichnet, dass man als Oxidationsmittel Mangandioxid, Chromtrioxid, ein Chromat oder, vorzugsweise, Aceton in Gegenwart eines Aluminiumalkoxids verwendet.

3. Verfahren gemäss Anspruch 2, dadurch gekennzeichnet, dass man als Oxidationsmittel Aceton in Gegenwart von Aluminiumisopropoxid oder Aluminium-t-butoxid verwendet.

4. Verfahren gemäss einem der Ansprüche 1 bis 3, dadurch gekennzeichnet, dass man das Auminiumalkoxid in einer Menge von etwa 0,1 bis etwa 100 Molprozent verwendet.

5. Verfahren gemäss einem der Ansprüche 1 bis 4, dadurch gekennzeichnet, dass man die Oxidation bei der Rückflusstemperatur des Reaktionsgemisches durchführt.

6. Verfahren gemäss einem der Ansprüche 1 bis 5, dadurch gekennzeichnet, dass man die Hydrierung mit einem teilweise vergifteten Palladium-Katalysator, vorzugsweise einem Lindlar-Katalysator durchführt.

7. Verfahren gemäss einem der Ansprüche 1 bis 6, dadurch gekennzeichnet, dass man die Hydrierung bei einer Temperatur von etwa 10°C bis etwa 70°C durchführt.

**Claims**

for the Contracting States: BE, CH, DE, FR, GB, IT, NL

1. Process for the manufacture of cyclohexene derivatives, characterized by reacting the compound of the formula

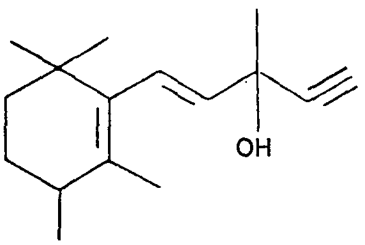
I

with an oxidation agent and, if desired, selectively hydrogenating the thus-obtained compound of the formula

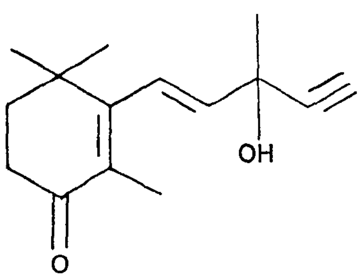
II

to the compound of the formula

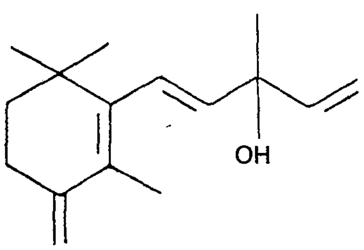
III

2. Process according to claim 1, characterized in that manganese dioxide, chromium trioxide, a chromate or, preferably, acetone in the presence of an aluminium alkoxide is used as the oxidation agent.

3. Process according to claim 2, characterized in that acetone in the presence of aluminium isopropoxide or aluminium t-butoxide is used as the oxidation agent.

4. Process according to one of claims 1 to 3, characterized in that the aluminium alkoxide is used in an amount of about 0.1 to about 100 mol percent.

5. Process according to one of claims 1 to 4, characterized in that oxidation is carried out at the reflux temperature of the reaction mixture.

6. Process according to one of claims 1 to 5, characterized in that the hydrogenation is carried out with a partially poisoned palladium catalyst, preferably a Lindlar catalyst.

7. Process according to one of claims 1 to 6, characterized in that the hydrogenation is carried out at a temperature of about 10°C to about 70°C.

8. Compound of the formula

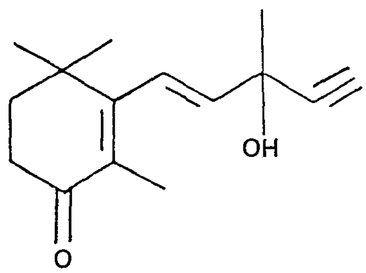
II

**Claims**

for the Contracting State: AT

1. Process for the manufacture of cyclohexene derivatives, characterized by reacting the compound of the formula

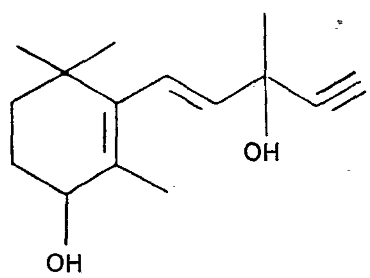
I

with an oxidation agent and, if desired, selectively hydrogenating the thus-obtained compound of the formula

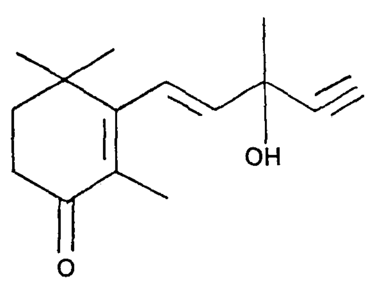
II

to the compound of the formula

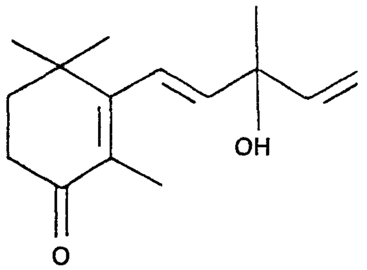

III

2. Process according to claim 1, characterized in that manganese dioxide, chromium trioxide, a chromate or, preferably, acetone in the presence of an aluminium alkoxide is used as the oxidation agent.

3. Process according to claim 2, characterized in that acetone in the presence of aluminium isopropoxide or aluminium t-butoxide is used as the oxidation agent.

4. Process according to one of claims 1 to 3, characterized in that the aluminium alkoxide is used in an amount of about 0.1 to about 100 mol percent.

5. Process according to one of claims 1 to 4, characterized in that the oxidation is carried out at the reflux temperature of the reaction mixture.

6. Process according to one of claims 1 to 5, characterized in that the hydrogenation is carried out with a partially poisoned palladium catalyst, preferably a Lindlar catalyst.

7. Process according to one of claims 1 to 6, characterized in that the hydrogenation is carried out at a temperature of about 10°C to about 70°C.

**Revendications**

pour l'Etats contractants: BE, CH, DE, FR, GB, IT, NL

1. Procédé de préparation de dérivés cyclohexéniques caractérisé en ce que l'on fait réagir le composé de formule:

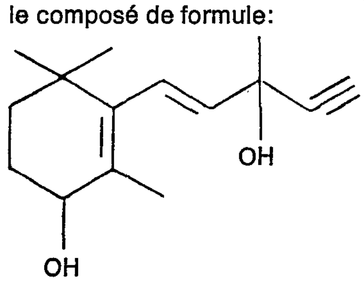

I

avec un agent oxydant, et, si on le désire, on soumet le composé obtenu de formule

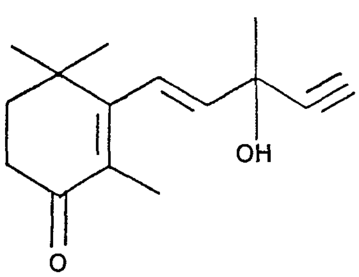

II

à hydrogénation sélective conduisant au composé de formule:

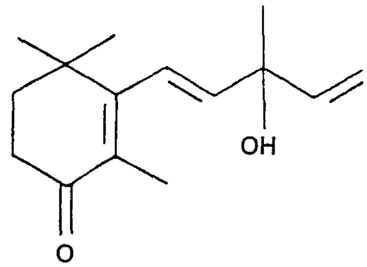

III

2. Procédé selon la revendication 1, caractérisé en ce que l'on utilise en tant qu'agent oxydant le bioxyde de manganèse, le trioxyde de chrome, un chromate, ou, de préférence, l'acétone en présence d'un alcoolate d'aluminium.

3. Procédé selon la revendication 2, caractérisé en ce que l'on utilise en tant qu'agent oxydant l'acétone en présence d'isopropylate d'aluminium ou de tert-butylate d'aluminium.

4. Procédé selon l'une des revendications 1 à 3, caractérisé en ce que l'on utilise l'alcoolate d'aluminium en quantité d'environ 0,1 à environ 100 moles pourcent.

5. Procédé selon l'une des revendications 1 à 4, caractérisé en ce que l'on effectue l'oxydation à la température de reflux du mélange de réaction.

6. Procédé selon l'une des revendications 1 à 5, caractérisé en ce que l'on effectue l'hydrogénation avec un catalyseur au palladium partiellement empoisonné, de préférence un catalyseur de Lindlar.

7. Procédé selon l'une des revendications 1 à 6, caractérisé en ce que l'on effectue l'hydrogénation à une température d'environ 10°C à environ 70°C.

8. Composé de formule:

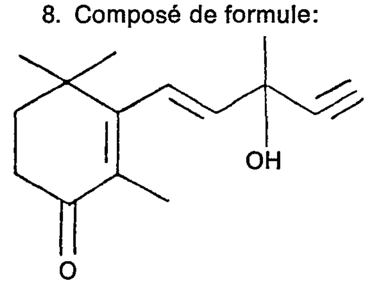

II

**Revendications**

pour l'Etat contractant: AT

1. Procédé de préparation de dérivés cyclohexéniques caractérisé en ce que l'on fait reagir le composé de formule:

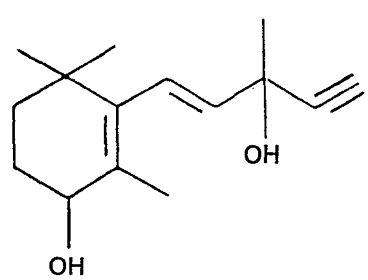

I

avec un agent oxydant, et, si on le désire, on soumet le composé obtenu de formule

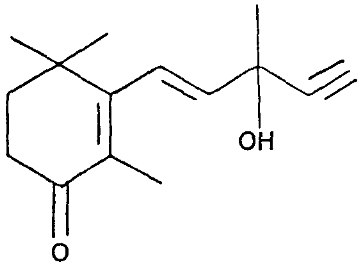

II

à hydrogénation sélective conduisant au composé de formule:

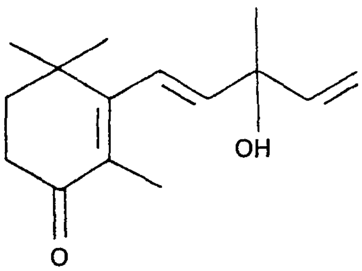

III

2. Procédé selon la revendication 1, caractérisé en ce que l'on utilise en tant qu'agent oxydant le bioxyde de manganèse, le trioxyde de chrome, un chromate, ou, de préférence, l'acétone en présence d'un alcoolate d'aluminium.

3. Procédé selon la revendication 2, caractérisé en ce que l'on utilise en tant qu'agent oxydant l'acétone en présence d'isopropylate d'aluminium ou de tert-butylate d'aluminium.

4. Procédé selon l'une des revendications 1 à 3, caractérisé en ce que l'on utilise l'alcoolate d'aluminium en quantité d'environ 0,1 à environ 100 moles pourcent.

5. Procédé selon l'une des revendications 1 à 4, caractérisé en ce que l'on effectue l'oxydation à la température de reflux du mélange de réaction.

6. Procédé selon l'une des revendications 1 à 5, caractérisé en ce que l'on effectue l'hydrogénation avec un catalyseur au palladium partiellement empoisonné, de préférence un catalyseur Lindlar.

7. Procédé selon l'une des revendications 1 à 6, caractérisé en ce que l'on effectue l'hydrogénation à une température d'environ 10°C à environ 70°C.